# EUROPEAN PATENT APPLICATION

(11) **EP 4 431 589 A1**
(43) Date of publication of application: **18.09.2024**
(21) Application number: 22890438.9
(22) Date of filing: 04.11.2022
(51) Int. Cl.: C12M 1/00, C12M 1/12, C12M 3/00

(54) **BIOREACTOR FOR ADHERENT CELLS**

(30) Priority: 08.11.2021 KR 20210152188
(71) Applicant: Amogreentech Co., Ltd., Gimpo-si, Gyeonggi-do 10014 (KR)
(72) Inventor: HAN, Kyoung Ku, Gimpo-si, Gyeonggi-do 10014 (KR); NOH, Hyeong Tak, Gimpo-si, Gyeonggi-do 10014 (KR); SONG, Jae Kyung, Gimpo-si, Gyeonggi-do 10014 (KR)
(74) Representative: Laine IP Oy
(86) International application number: PCT/KR2022/017233
(87) International publication number: WO 2023/080707

(57) **Abstract**

Provided is a bioreactor for adherent cells. The bioreactor for adherent cells according to one embodiment of the present invention comprises: a housing including a housing body, which has an enclosed shape and has an inner space open on one side, and a cover detachably coupled to the housing body so as to cover the open side of the inner space; a support assembly including a plurality of supports, which are formed as plate-shaped members having a predetermined area so that cells to be cultured can be attached to at least one surface, and which are disposed at intervals in multiple stages along one direction, and a plurality of fastening bars fastening the plurality of supports; and a pair of guide grooves which are formed recessed in the inner surface of the housing to guide the sliding of the support assembly so that a physical impact can be applied to the support assembly when the support assembly is moved by an external force.

## Description

### Technical field

The present invention relates to a bioreactor for adherent cells.

### Background Art

Cell culture refers to isolating cells from multicellular organisms and proliferating them in vitro.

Cultured cells may be classified into adherent culture cells and suspension culture cells depending on their form of existence.

Among these, adherent culture cells proliferate while attached to the surface of a support. When culture is completed, these adherent culture cells are chemically separated from the support using a chemical substance such as trypsin and recovered.

However, since chemical substances such as trypsin interfere with the action of cell adhesion proteins and cause cells to separate from the support, there is a risk that cell proteins may be destroyed when cells are exposed to a chemical substance such as trypsin for a long time. On the other hand, when the exposure time to a chemical substance such as trypsin is shortened to prevent cell protein destruction, there is a problem in that the recovery rate of cells decreases.

Therefore, there is a need for a method that can prevent cell destruction while increasing the recovery rate of cells after completion of culture.

### Disclosure

### Technical Problem

The present invention was devised in view of the above points, and the purpose is to provide a bioreactor for adherent cells that may increase the recovery rate of cells after completion of culture by separating cells attached to a support through physical impact.

### Technical Solution

In order to solve the above problems, the present invention provides a bioreactor for adherent cells including: a housing including a housing body in a box shape having an inner space with one open surface, and a cover detachably coupled to the housing body so as to cover the one open surface of the inner space; a support assembly including a plurality of supports formed of a plate-shaped member with a predetermined area so that cells to be cultured are attached to at least one side and disposed in multiple stages at intervals along one direction, and a plurality of fastening bars fastening the plurality of supports; and a pair of guide grooves formed by recessing an inner surface of the housing to guide the sliding movement of the support assembly when the support assembly is moved by an external force so that a physical impact is applied to the support assembly.

In addition, the pair of guide grooves may include a first guide groove formed in a bottom surface of the housing body facing one surface of the cover, and a second guide groove formed in one surface of the cover, and each of the first guide groove and the second guide groove may be long hole guide grooves formed at positions corresponding to each other so that both ends of the fastening bar are inserted thereinto, respectively.

In addition, the housing may include an inlet for supplying a medium to the inner space and an outlet for discharging the medium in the inner space to the outside, and the long hole guide groove may be formed so that a first width in a direction perpendicular to an imaginary straight line connecting the inlet and the outlet is wider than a second width in a direction parallel to the imaginary straight line.

In this case, the support assembly is capable of sliding in both directions along the first width direction through the long hole guide groove in the inner space, and the support assembly may be restricted in movement in the second width direction through the long hole guide groove.

In addition, the support assembly may further include a first plate and a second plate having a predetermined area, and a spacing member disposed between two supports with one surface facing each other, the plurality of supports may be disposed between the first and second plates to maintain a certain distance through the spacing member while one surface thereof faces each other, and the first plate, the second plate, the plurality of supports, and the spacing member may be integrated through the plurality of fastening bars.

In addition, the support may include a corresponding portion facing the first plate or the second plate, and a protruding portion extending from the corresponding portion so as to protrude a certain length outward from an edge of the first plate or the second plate, and the protruding portion may come into contact with an inner surface of the housing body when the support assembly is slidably moved.

In addition, the bioreactor for adherent cells may include a protruding bar that protrudes a certain length from one surface of the cover into the inner space, the support assembly may include a placement hole formed through the plurality of supports along a stacking direction, and the protruding bar may be inserted and disposed into the placement hole while the support assembly is accommodated in the inner space.

In addition, the placement hole may be formed to have a size larger than the thickness of the protruding bar.

In addition, a circumferential surface of the protruding bar may come into contact with an edge of the placement hole when the support assembly is slidably moved.

In addition, the protruding bar may be disposed such that one end is fixed to the cover and the other end is not fixed to a bottom surface of the housing body.

In addition, the support may include a plate-shaped nanofiber membrane or be a plate-shaped film member.

### Advantageous Effects

According to the present invention, cells attached to a support can be smoothly separated by transmitting a physical shock to the support to which the cells are attached or by applying a direct impact to the support. Through this, the present invention can increase the recovery rate of cells after completion of culture.

### Brief Description of Drawings

FIG. 1 is a schematic diagram showing a bioreactor for adherent cells according to one embodiment of the present invention.
FIG. 2 is a view of FIG. 1 viewed from another direction.
FIG. 3 is a view in which the main components in FIG. 1 are separated.
FIG. 4 is a cross-sectional view taken in the A-A direction of FIG. 1.
FIG. 5 is a cross-sectional view taken in the B-B direction of FIG. 1.
FIG. 6 is a view in which the support assembly in FIG. 3 is taken out and separated.
FIGS. 7 and 8 are views showing a state in which the support assembly in FIG. 4 is slidably moved along a long hole guide groove.
FIG. 9 is a schematic diagram showing a bioreactor for adherent cells according to another embodiment of the present invention.
FIG. 10 is a view of FIG. 9 viewed from another direction.
FIG. 11 is a view in which the main components in FIG. 9 are separated
FIG. 12 is a cross-sectional view taken in the C-C direction of FIG. 9.
FIG. 13 is a cross-sectional view taken in the D-D direction of FIG. 9.
FIG. 14 is a view in which the support assembly in FIG. 11 is taken out and separated.
FIGS. 15 and 16 are views showing a state in which the support assembly in FIG. 12 is slidably moved along a long hole guide groove.

### Modes of the Invention

Hereinafter, embodiments of the present invention will be described in detail with reference to the accompanying drawings so as to be easily carried out by those skilled in the art. The present invention may be embodied in many different forms and is not limited to the embodiments set forth herein. In order to clearly describe the present invention in the drawings, portions that are irrelevant to the description are omitted, and the same reference numerals are added to the same or similar elements throughout the specification.

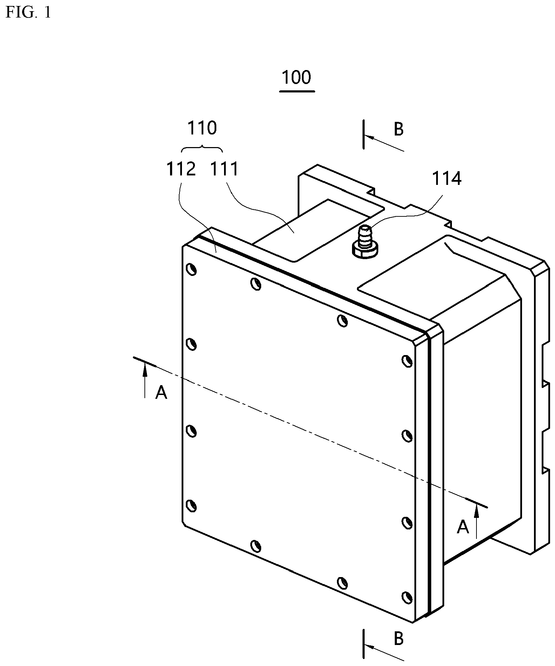

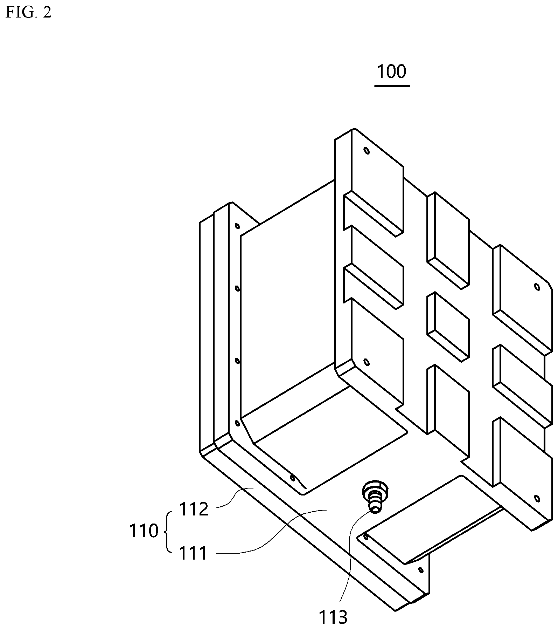

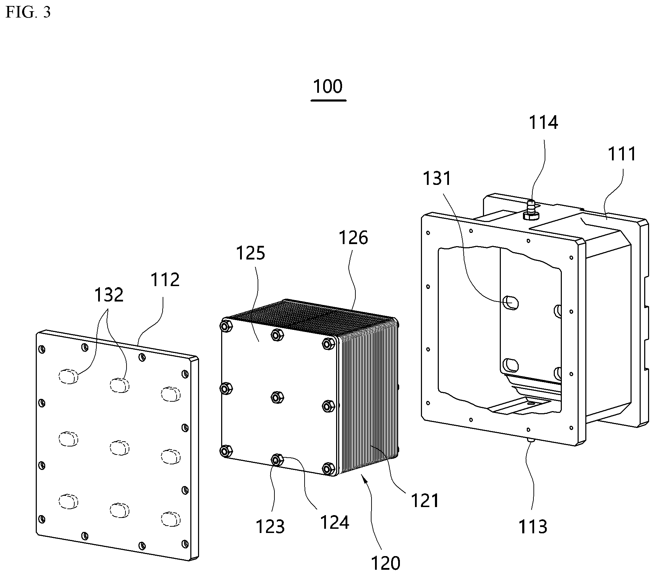

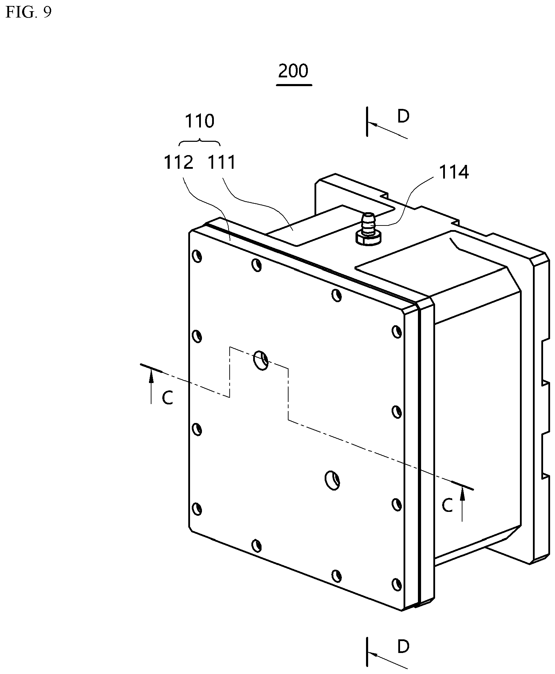

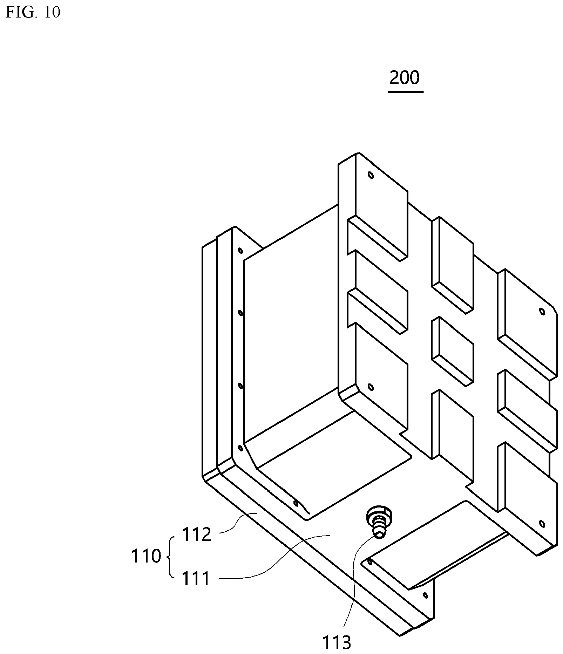

The bioreactor for adherent cells 100 or 200 according to one embodiment of the present invention includes a housing 110, a support assembly 120 or 220, and a pair of guide grooves 131 and 132 as shown in FIGS. 1 to 3 and 9 to 11.

The housing 110 may accommodate the support assembly 120 or 220 and a medium for cell culture therein. To this end, the housing 110 may be formed in a box shape with an inner space.

For example, the housing 110 may include a housing body 111 in a box shape having an inner space with one open side and a cover 112 covering the one open side of the housing body 111.

In this case, the housing 110 may include one or more ports 113 and 114 formed on one side of the housing body 111, and the one or more ports 113 and 114 may introduce a medium required for cell culture from the outside into the inner space or discharge the medium from the inner space to the outside. For example, the one or more ports 113 and 114 may include an inlet 113 for supplying a medium from the outside to the inner space and an outlet 114 for discharging the medium in the inner space to the outside, and the inlet 113 and outlet 114 may be formed on both sides of the housing body 111, respectively.

Through this, the inner space may be filled with a medium supplied from the outside through the inlet 113, and the medium filled in the inner space may be discharged to the outside through the outlet 114.

In this case, the medium may be circulated to be discharged from the inner space to the outside through the outlet 114 while moving into the inner space through the inlet 113.

However, the present invention is not limited thereto, and the port may be provided as one port, and the one port may serve both as an inlet for supplying a medium from the outside to the inner space and as an outlet for discharging the medium from the inner space to the outside after cell culture is completed.

The support assembly 120 or 220 may include a plurality of supports 121 and may be disposed in the inner space.

Herein, the support 121 may provide a culture surface to which cells to be cultured are attached.

To this end, as the support 121, various materials known to be used in cell culture may be used without limitation as long as it may be implemented in a plate-like form and cells may be easily attached thereto.

As a non-limiting example, the support 121 may include a nanofiber membrane in which nanofibers are formed into a three-dimensional network structure through electrospinning. Alternatively, the support 121 may be a plate-shaped film member.

In addition, the support 121 may have a modified surface so that the cells to be cultured may be smoothly attached to the surface. That is, the nanofiber membrane may be a membrane whose surface is coated with a motif, and the plate-shaped film member may be a film member whose surface is coated with a motif.

At this time, the plurality of supports 121 may be disposed so that one surface facing each other along one direction is spaced at a certain distance.

Accordingly, cells to be cultured may be attached in large quantities to the plurality of supports 121, and cells to be cultured can be cultured in large quantities through nutrients supplied from the medium while attached to each support 121,

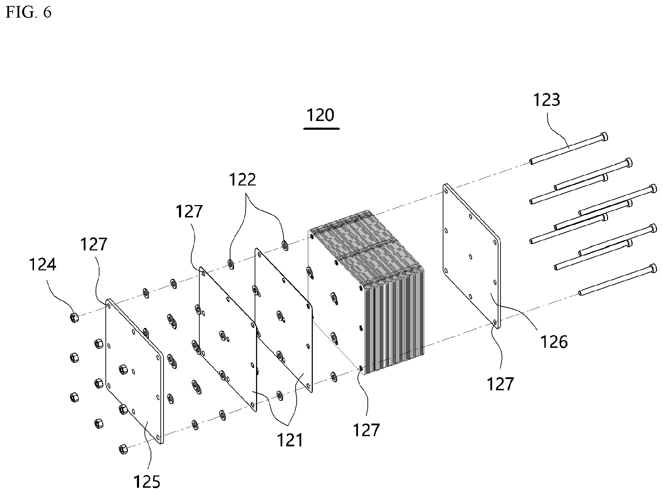

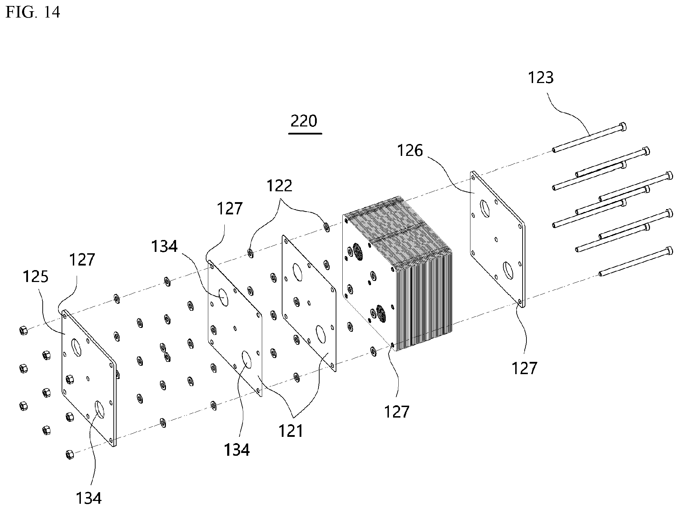

To this end, the support assembly 120 or 220 may include a plate-shaped first plate 125 and a second plate 126 having a predetermined area, as shown in FIGS. 6 and 14, a spacing member 122 disposed between two supports 121 with one surface facing each other, and a plurality of fastening bars 123 having a predetermined length.

In this case, the plurality of supports 121 may be disposed so that one surface faces between the first plate 125 and the second plate 126, a gap between the two supports 121 with one surface facing each other may be maintained through the spacing member 122, and the first plate 125, the second plate 126, the plurality of supports 121, and the spacing member 122 may be integrated through the fastening bar 123.

That is, the plurality of fastening bars 123 may be spaced apart from each other at a predetermined distance, both ends of the plurality of fastening bars 123 may be fixed to the first plate 125 and the second plate 126, respectively, and the plurality of supports 121 may be respectively fastened to the fastening bars 123 through a plurality of fastening holes 127 formed through positions corresponding to the plurality of fastening bars 123.

In addition, the spacing members 122 may be respectively fitted onto the plurality of fastening bars 123 like the support 121, and the plurality of spacing members 122 and the plurality of supports 121 may be alternately fastened to each fastening bar 123.

Accordingly, the spacing members 122 may be respectively disposed between two supports 121 arranged along one direction, two supports 121 with one surface facing each other may maintain a gap through the spacing member 122, and the first plate 125, the second plate 126, the plurality of supports 121, and the spacing members 122 may be integrated through a plurality of fastening bars 123.

Herein, a first fixing member 124, such as a nut member, may be detachably coupled to one end of the fastening bar 123, and the first fixing member 124 may be fastened to an end of the fastening bar 123 in a state where the first plate 125, the plurality of supports 121, the spacing members 122, and the second plate 126 are fastened to the fastening bar 123.

At this time, in the bioreactor for adherent cells 100 or 200 according to one embodiment of the present invention, when an external force is applied to the housing 110 while the support assembly 120 or 220 is accommodated in the inner space, the support assembly 120 or 220 may be slidably moved in the inner space.

Accordingly, the amount of impact generated when sliding the support assembly 120 or 220 may be transmitted to the cells attached to the support 121.

For this reason, the bioreactor for adherent cells 100 or 200 according to one embodiment of the present invention may easily separate cells that have completed culturing while attached to the support 121 from the support 121.

That is, because the bioreactor for adherent cells 100 or 200 according to one embodiment of the present invention may apply a physical impact to the adherent cells attached to the support 121 through the sliding movement of the support assembly 120 or 220, the adherent cells may be separated from the support 121 through a physical method.

Through this, in the bioreactor for adherent cells 100 or 200 according to one embodiment of the present invention, the adherent cells attached to the support 121 may be separated from the support 121 using both a chemical separation method using a chemical substance such as trypsin and a physical separation method using a physical impact.

Through this, when the bioreactor for adherent cells 100 or 200 according to one embodiment of the present invention recovers the adherent cells after completion of culture from the support 121, because the use time of the chemical substance used to separate cells after completion of culture from the support 121 can be reduced, damage to cells caused by the chemical substance may be minimized.

In addition, because the bioreactor for adherent cells 100 or 200 according to one embodiment of the present invention may separate adherent cells from the support 121 by utilizing both a chemical method using a chemical substance and a physical method using a physical impact, the recovery rate of the adherent cells after completion of culture may be further increased.

For this reason, the bioreactor for adherent cells 100 or 200 according to one embodiment of the present invention is capable of culturing a large amount of cells through a plurality of supports 121, and since cells after completion of culture may be smoothly separated from each support 121 to increase the recovery rate, a greater amount of cells may be obtained through a single process.

To this end, the bioreactor for adherent cells 100 or 200 according to one embodiment of the present invention may include a pair of guide grooves 131 and 132, which are formed by recessing the inner surface of the housing 110, to guide the sliding movement of the support assembly 120 or 220 while the support assembly 120 or 220 is accommodated in the inner space.

For example, the pair of guide grooves 131 and 132 may include a first guide groove 131 formed by recessing the bottom surface of the housing body 111 facing one surface of the cover 112 to a certain depth and a second guide groove 132 formed by recessing one surface of the cover 112 to a certain depth.

In this case, each of the first guide groove 131 and the second guide groove 132 may be long hole guide grooves formed at positions corresponding to each other so that both ends of the fastening bar 123 can be respectively inserted thereinto, the first guide groove 131 and the second guide groove 132 may be formed to have shapes corresponding to each other, and each of the first guide groove 131 and the second guide groove 132 may be formed to have both ends sealed.

In addition, the long hole guide groove may be formed so that first widths W1 and W3 in a direction perpendicular to an imaginary straight line connecting the inlet 113 and the outlet 114 is wider than second widths W2 and W4 in a direction parallel to the imaginary straight line.

That is, the long hole guide groove may be formed so that the length in the direction parallel to the imaginary straight line connecting the inlet 113 and the outlet 114 is longer than the length in the direction perpendicular to the imaginary straight line.

At this time, the second widths W2 and W4 may be widths that restrain the movement of the fastening bar 123 along the direction of the second widths W2 and W4 while accommodating both ends of the fastening bar 123.

Accordingly, both ends of the fastening bar 123 may be slidably moved in both directions along a direction parallel to the first widths W1 and W3 within the first guide groove 131 and the second guide groove 132, the movement in a direction parallel to the direction of the second widths W2 and W4 may be restricted, and the linear movement distance of the fastening bar 123 may be restricted through the both sealed ends of the first guide groove 131 and the second guide groove 132, respectively.

Through this, when an external force is applied to the housing 110, the support assembly 120 or 220 may be slidably moved in a direction parallel to the first widths W1 and W3 within the inner space through the fastening bar 123 that is slidably moved along the first guide groove 131 and the second guide groove 132, and the moving distance may be restricted through the both sealed ends of the first guide groove 131 and the second guide groove 132, respectively.

In addition, the support assembly 120 or 220 may be restricted from sliding in a direction parallel to the second widths W2 and W4 through the first guide groove 131 and the second guide groove 132 regardless of whether there is an external force applied to the housing 110.

For example, when an operator shakes the housing 110 in both directions along a direction parallel to the first widths W1 and W3, the support assembly 120 or 220 may be slidably moved in a direction parallel to the first widths W1 and W3 within the inner space through the fastening bar 123 that is slidably moved along the first guide groove 131 and the second guide groove 132.

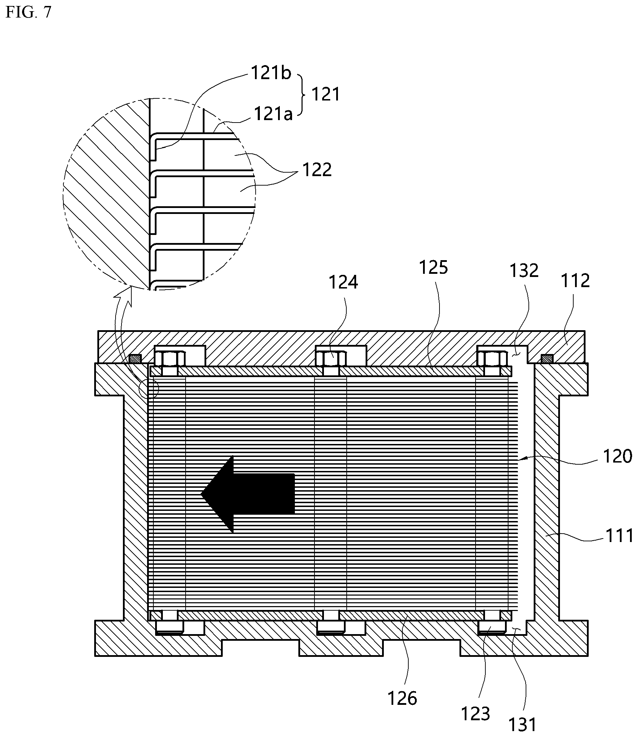

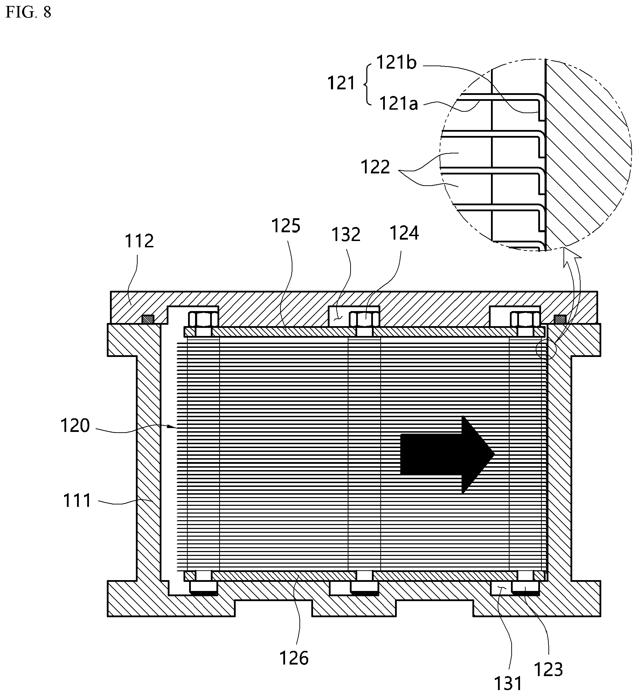

Through this, as shown in FIGS. 7, 8, 15, and 16, when both ends of the fastening bar 123 move toward one of the both sealed ends of the first guide groove 131 and the second guide groove 132, both ends of the fastening bar 123 come into contact with the sealed ends of the first guide groove 131 and the second guide groove 132, and thus an impact may be applied to the support assembly 120 or 220.

Due to this, the impact applied to the support assembly 120 or 220 may be transmitted to the support 121, and the adherent cells attached to the support 121 may be smoothly separated from the support 121 through an impact applied to the support 121.

In this way, because the bioreactor for adherent cells 100 or 200 according to one embodiment of the present invention may apply a physical impact to the adherent cells attached to the support 121 through the sliding movement of the support assembly 120 or 220, the adherent cells may be separated from the support 121 through a physical method.

In addition, even when the housing 110 is disposed with the inlet 113 facing downward in order to culture the cells attached to the support 121, the support assembly 120 or 220 disposed in the inner space may be restricted from sliding in a direction parallel to the second widths W2 and W4 through the first guide groove 131 and the second guide groove 132.

Accordingly, in the support assembly 120 or 220, each support 121 may be maintained while disposed parallel to an imaginary straight line connecting the inlet 113 and the outlet 114 without moving along the direction of the second widths W2 and W4, and adherent cells to be cultured may be cultured while attached to each support 121 disposed parallel to the imaginary straight line without moving along the direction of the second widths W2 and W4.

At this time, in the bioreactor for adherent cells 100 or 200 according to one embodiment of the present invention, a physical impact generated during the sliding movement of the support assembly 120 or 220 guided through the first guide groove 131 and the second guide groove 132 may be directly applied to the support 121.

Through this, in the bioreactor for adherent cells 100 or 200 according to one embodiment of the present invention, adherent cells attached to the support 121 may be separated from the support 121 using both indirect physical shock transmitted from the fastening bar 123 when sliding the support assembly 120 or 220, and direct physical impacts generated when sliding the support assembly 120 or 220.

To this end, in the bioreactor for adherent cells 100 or 200 according to one embodiment of the present invention, the support 121 may be provided to have a larger area than the first plate 125 and the second plate 126.

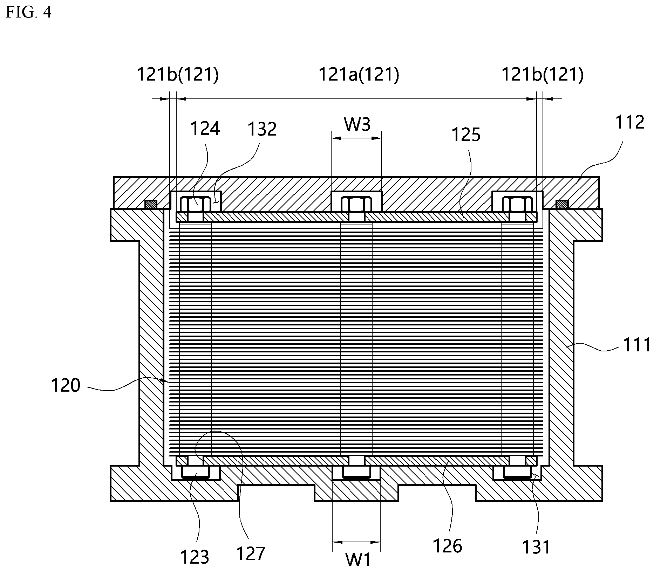

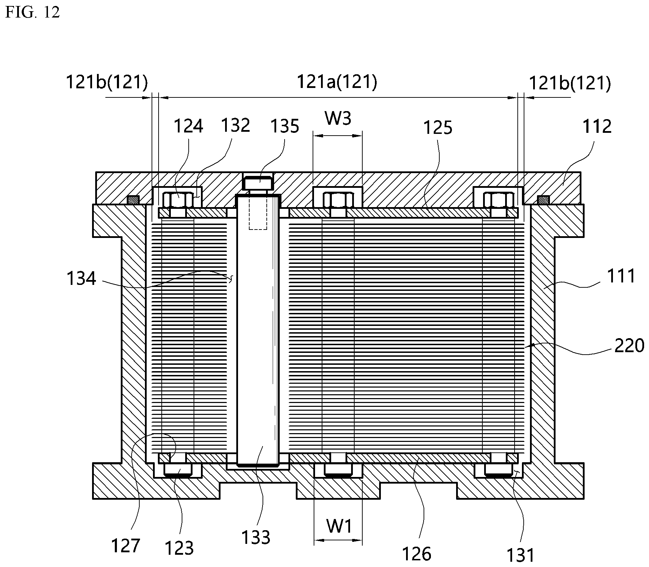

That is, as shown in FIGS. 4 and 12, the support 121 includes a corresponding portion 121a facing the first plate 125 or the second plate 126, and a pair of protruding portions 121b each extending from the corresponding portion 121a so as to protrude a certain length outward from the edge of the first plate 125 or the second plate 126.

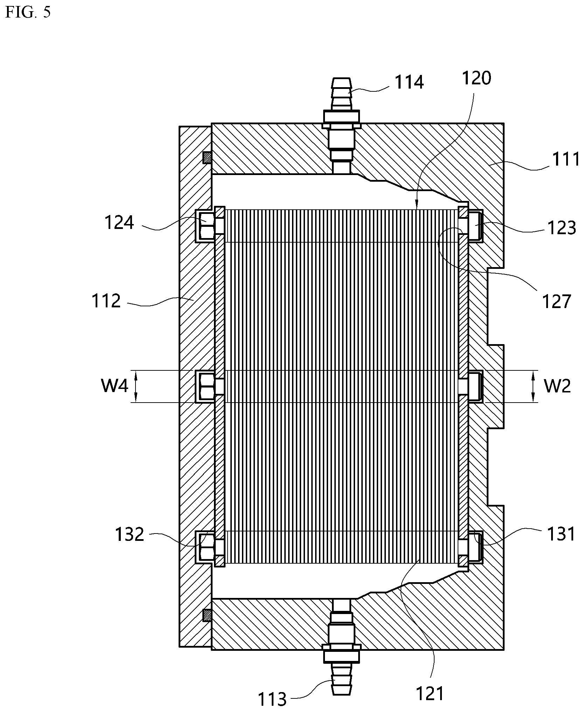

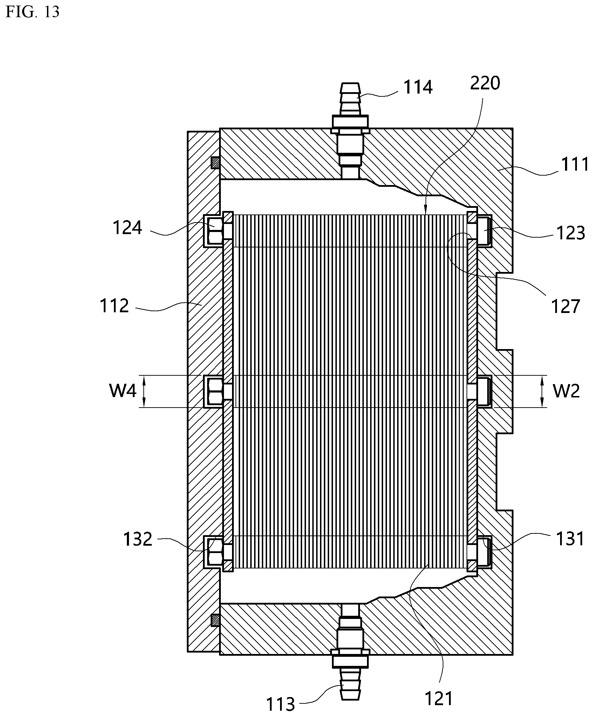

In this case, the pair of protruding portions 121b may each extend from the corresponding portion 121a in a direction parallel to the direction of the first widths W 1 and W3 of the first guide groove 131 and the second guide groove 132, and as shown in FIGS. 5 and 13, the pair of protruding portions 121b may not extend from the corresponding portion 121a in a direction parallel to the direction of the second widths W2 and W4 of the first guide groove 131 and the second guide groove 132.

Through this, as shown in enlarged views of FIGS. 7, 8, 15, and 16, when both ends of the fastening bar 123 move toward one of the both sealed ends of the first guide groove 131 and the second guide groove 132, one of the pair of protruding portions 121b may be bent when coming into contact with the inner surface of the housing body 111, and a direct physical impact may be applied to the support 121 through the contact between the protruding portion 121b and the housing body 111.

As another example, the bioreactor for adherent cells 200 according to one embodiment of the present invention may include at least one protruding bar 133 that protrudes a certain length from one surface of the cover 112 into the inner space where the support assembly 220 is accommodated.

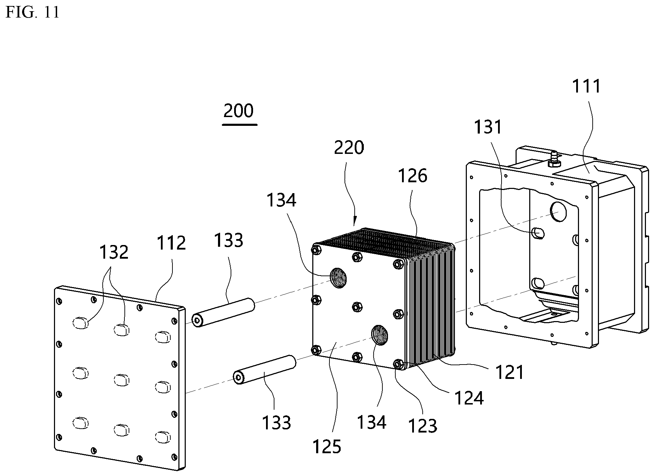

In this case, the support assembly 220 may include a placement hole 134 formed through a predetermined area along the stacking direction of the plurality of supports 121, as shown in FIGS. 11 and 14.

Accordingly, as shown in FIG. 12, the protruding bar 133 may be inserted and disposed into the placement hole 134 while the support assembly 220 is disposed in the inner space.

At this time, one end of the protruding bar 133 may be fixed to the cover 112, and the other end may not be fixed to the bottom surface of the housing body 111. That is, one end of the protruding bar 133 may be detachably coupled to the cover 112 through a second fixing member 135 such as a bolt member, and the protruding bar 133 may be inserted and disposed into the placement hole 134 of the support assembly 220 with one end fixed to the cover 112.

In addition, the width of the placement hole 134 may be formed to be larger than the thickness of the protruding bar 133.

Accordingly, even when the support assembly 220 is slidably moved along the first guide groove 131 and the second guide groove 132, the protruding bar 133 may maintain a fixed position without changing its position in the inner space where the support assembly 220 is accommodated.

Through this, as shown in FIG. 12, when both ends of the fastening bar 123 in the support assembly 220 do not come into contact with the both sealed ends of the first guide groove 131 and the second guide groove 132, the protruding bar 133 may be disposed in the placement hole 134 with its circumferential surface not in contact with the edge of the placement hole 134.

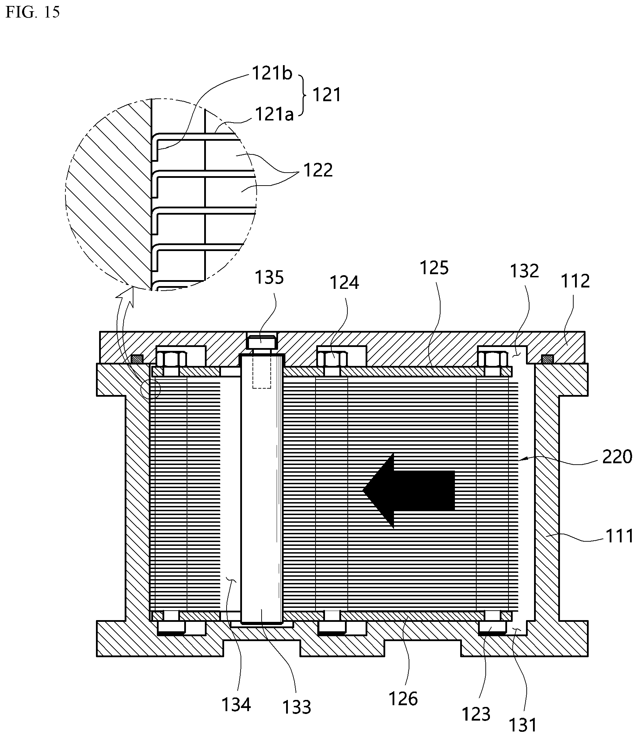

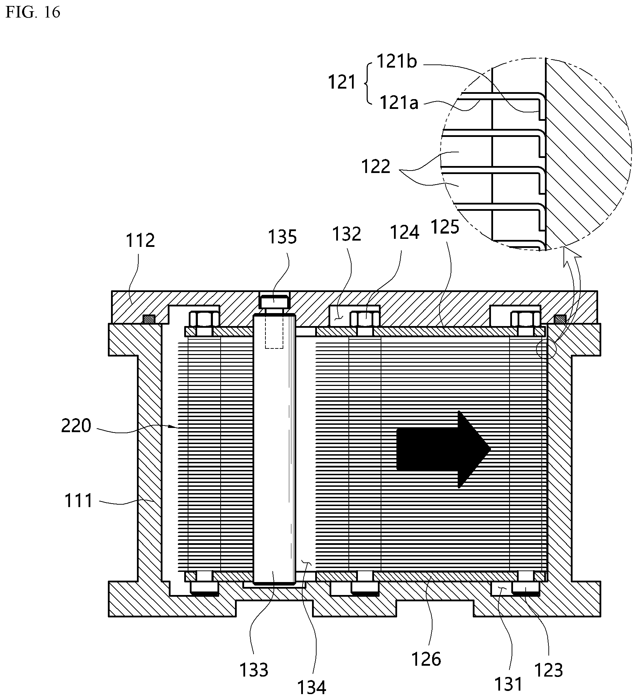

On the other hand, as shown in FIGS. 15 and 16, when an external force is applied to the housing 110 while the support assembly 220 is accommodated in the inner space, the support assembly 220 may be slidably moved along the first guide groove 131 and the second guide groove 132 in a state in which the position of the protruding bar 133 is fixed.

Accordingly, when both ends of the fastening bar 123 move toward one of the both sealed ends of the first guide groove 131 and the second guide groove 132 in a state in which the position of the protruding bar 133 is fixed, the circumferential surface of the protruding bar 133 may come into contact with an edge defining the placement hole 134.

Through this, a direct physical impact may be applied to the support 121 through the contact between the protruding bar 133 and the edge of the placement hole 134.

Through this, in the bioreactor for adherent cells 100 or 200 according to one embodiment of the present invention, indirect physical shock generated through contact between the fastening bar 123 and the first guide groove 131 and the second guide groove 132 when sliding the support assemblies 120 and 220, and a direct physical impact generated through contact between the pair of protruding portions 121b and the housing body 111 and/or contact between the circumferential surface of the protruding bar 133 and the edge of the placement hole 134 when sliding the support assembly 120 or 220 may be applied to the support assembly 120 or 220.

As a result, since both indirect physical shock and direct physical impacts can be applied to the adherent cells attached to the support 121, the adherent cells attached to the support 121 may be smoothly separated from the support 121.

Through this, the bioreactor for adherent cells 100 or 200 according to one embodiment of the present invention may increase the recovery rate of adherent cells because a greater amount of adherent cells can be separated from the support 121 while easily separating adherent cells after completion of culture from the support 121.

In the above description, as a method for applying a physical impact to the support assembly 120 or 220, although it has been described that a pair of guide grooves 131 and 132 and a pair of protruding portions 121b are applied or a pair of guide grooves 131 and 132, a protruding bar 133 and a placement hole 134 are applied, the present invention is not limited thereto, and a pair of guide grooves 131 and 132, a pair of protruding portions 121b, a protruding bar 133, and a placement hole 134 may all be applied.

Although one embodiment of the present invention has been described above, the spirit of the present invention is not limited to the embodiment presented herein, and those skilled in the art and having an understanding of the scope of the invention will readily suggest other embodiments by altering, deleting, adding, etc., components within the same spirit, which are also intended to fall within the spirit of the present invention.

## Claims

1. A bioreactor for adherent cells comprising:
a housing including a housing body in a box shape having an inner space with one open surface, and a cover detachably coupled to the housing body so as to cover the one open surface of the inner space;
a support assembly including a plurality of supports formed of a plate-shaped member with a predetermined area so that cells to be cultured are attached to at least one surface and disposed in multiple stages at intervals along one direction, and a plurality of fastening bars fastening the plurality of supports; and
a pair of guide grooves formed by recessing an inner surface of the housing and configured to guide the sliding movement of the support assembly when the support assembly is moved by an external force so that a physical impact is applied to the support assembly.

2. The bioreactor for adherent cells of claim 1, wherein the pair of guide grooves include a first guide groove formed in a bottom surface of the housing body facing one surface of the cover, and a second guide groove formed in one surface of the cover, and
each of the first guide groove and the second guide groove is long hole guide grooves formed at positions corresponding to each other so that both ends of the fastening bar are inserted thereinto, respectively.

3. The bioreactor for adherent cells of claim 2, wherein the housing includes an inlet for supplying a medium to the inner space and an outlet for discharging the medium in the inner space to the outside,
the long hole guide groove is formed so that a first width in a direction perpendicular to an imaginary straight line connecting the inlet and the outlet is wider than a second width in a direction parallel to the imaginary straight line,
the support assembly is capable of sliding in both directions along the first width direction through the long hole guide groove in the inner space, and
the support assembly is restricted in movement in the second width direction through the long hole guide groove.

4. The bioreactor for adherent cells of claim 1, wherein the support assembly further includes a first plate and a second plate having a predetermined area and a spacing member disposed between two supports with one surface facing each other,
the plurality of supports are disposed between the first and second plates to maintain a certain distance through the spacing member while one surface thereof faces each other, and
the first plate, the second plate, the plurality of supports, and the spacing member are integrated through the plurality of fastening bars.

5. The bioreactor for adherent cells of claim 4, wherein the support includes a corresponding portion facing the first plate or the second plate, and a pair of protruding portions each extending from the corresponding portion so as to protrude a certain length outward from an edge of the first plate or the second plate, and
the pair of protruding portions come into contact with an inner surface of the housing body when the support assembly is slidably moved.

6. The bioreactor for adherent cells of claim 1, comprising a protruding bar that protrudes a certain length from one surface of the cover into the inner space,
wherein the support assembly includes a placement hole formed through the plurality of supports along a stacking direction, and
the protruding bar is inserted and disposed into the placement hole while the support assembly is accommodated in the inner space.

7. The bioreactor for adherent cells of claim 6, wherein the placement hole is formed to have a size larger than the thickness of the protruding bar.

8. The bioreactor for adherent cells of claim 6, wherein a circumferential surface of the protruding bar comes into contact with an edge of the placement hole when the support assembly is slidably moved.

9. The bioreactor for adherent cells of claim 6, wherein the protruding bar is disposed such that one end is fixed to the cover and the other end is not fixed to a bottom surface of the housing body.

10. The bioreactor for adherent cells of claim 1, wherein the support includes a plate-shaped nanofiber membrane or is a plate-shaped film member.
